(19) Europäisches Patentamt
European Patent Office
Office européen des brevets

(11) Veröffentlichungsnummer: **0 158 956**
**B1**

(12) # EUROPÄISCHE PATENTSCHRIFT

(45) Veröffentlichungstag der Patentschrift: **06.09.89**

(51) Int. Cl.⁴: **C 07 D 211/78**, A 61 K 31/44

(21) Anmeldenummer: **85104256.4**

(22) Anmeldetag: **09.04.85**

(54) **4-(Nitrophenyl)-tetrahydropyridine, Verfahren zu ihrer Herstellung und ihre Verwendung als Arzneimittel.**

(30) Priorität: **19.04.84 DE 3414801**

(43) Veröffentlichungstag der Anmeldung: **23.10.85 Patentblatt 85/43**

(45) Bekanntmachung des Hinweises auf die Patenterteilung: **06.09.89 Patentblatt 89/36**

(84) Benannte Vertragsstaaten: **AT BE CH DE FR GB IT LI NL SE**

(56) Entgegenhaltungen:
**EP-A- 0 110 073**
**EP-A- 0 125 803**
**US-A- 4 361 572**

**SYNTHESIS, Nr. 2, Februar 1985, Seiten 210-212 U. ROSENTRETER: "Stereoselective synthesis of all-trans-isomers of 1,2,3,4-tetrahydropyridines and piperidines from Hantzsch-type 1,4-dihydropyridines."**

(73) Patentinhaber: **BAYER AG, D-5090 Leverkusen 1 Bayerwerk (DE)**

(72) Erfinder: **Rosentreter, Ulrich, Dr., Kondorweg 23, D-5600 Wuppertal 1 (DE)**
Erfinder: **Thomas, Günther, Dr., Claudiusweg 9, D-5600 Wuppertal 1 (DE)**
Erfinder: **Knorr, Andreas, Dr., Pahlkestrasse 15, D-5600 Wuppertal 1 (DE)**

**EP 0 158 956 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die neue Erfindung betrifft neue 4-Nitrophenyltetrahydropyridine, ein Verfahren zu ihrer Herstellung sowie ihre Verwendung als Arzneimittel, insbesondere als kreislaufbeeinflussende Mittel.

Aus dem US-Patent 4 361 572 sind bereits Tetrahydropyridine bekannt, die sich jedoch sowohl in ihrer Struktur als auch in ihrer Wirkung eindeutig von den erfindungsgemäßen Verbindungen unterscheiden.

Die vorliegende Erfindung betrifft neue 4-(Nitrophenyl)-1,2,3,4-tetrahydropyridine der allgemeinen Formel (I)

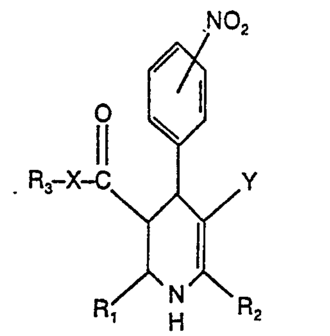
(I)

in welcher

$R_1$ und $R_2$ jeweils für Alkyl mit 1 bis 4 Kohlenstoffatomen stehen,

$R_3$ für einen geradkettigen oder verzweigten Alkylrest mit bis zu 10 Kohlenstoffatomen steht, der gegebenenfalls durch Sauerstoff in der Kette unterbrochen ist oder durch Fluor oder Chlor substituiert ist,

X für eine Einfachbindung oder ein Sauerstoffatom steht,

und

Y für eine Nitrilgruppe oder den Rest $-CO-X'-R_4$ steht, wobei X' und $R_4$ jeweils die Bedeutung von X und $R_3$ besitzt und mit diesen identisch oder von diesen verschieden sein kann,

sowie ihre physiologisch unbedenklichen Salze.

Es wurde gefunden, daß man die erfindungsgemäßen Verbindungen der Formel (I) erhält, wenn man Dihydropyridinverbindungen der allgemeinen Formel (II)

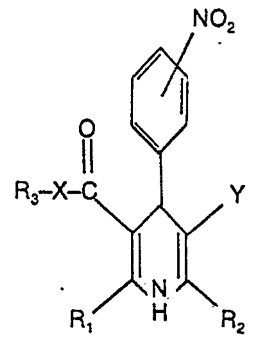
(II)

in welcher

$R_1$, $R_2$, $R_3$, Y und X die oben angegebene Bedeutung haben,

in einem stark sauren Reaktionsmedium mittels geeigneter Hydriddonoren reduziert.

Die erfindungsgemäßen 4-(Nitrophenyl)-1,2,3,4-tetrahydropyridinderivate besitzen wertvolle pharmakologische Eigenschaften. Aufgrund ihrer kreislaufbeeinflussenden Wirkung können sie als antihypertensive Mittel, als periphere und cerebrale Vasodilatatoren sowie als Coronartherapeutika Verwendung finden und sind somit als Bereicherung der Pharmazie anzusehen.

Die Darstellung der erfindungsgemäßen Verbindungen kann durch folgendes Formelschema wiedergegeben werden, wobei der 2,6-Dimethyl-4-(2-nitrophenyl)-1,2,3,4-tetrahydropyridin-3,5-dicarbonsäuredimethylester als Beispiel gewählt sei:

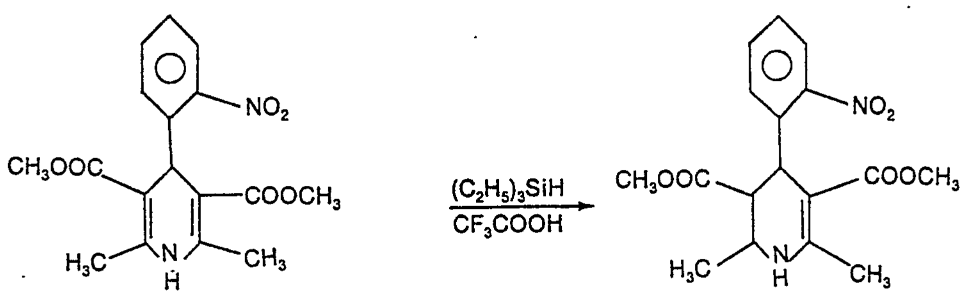


Gemäß diesem Verfahren wird die Reduktion der eingesetzten 1,4-Dihydropyridine in einem stark sauren Reaktionsmedium durchgeführt.

Als stark saure Reaktionsmedien seien vorzugsweise genannt:

Trifluoressigsäure, Mischungen von Trifluoressigsäure mit Essigsäure, Dichlormethan, Chloroform, Benzol, Toluol, 1,2-Dichlorethan, Dioxan, Tetrahydrofuran, Nitromethan und Acetonitril, Mischungen von Schwefelsäure mit Eisessig, Lösungen von Methansulfonsäure oder p-Toluolsulfonsäure in Dichlormethan, Chloroform, Benzol, Toluol, 1,2-Dichlorethan, Dioxan, Tetrahydrofuran, Nitromethan und Acetonitril, sowie Mischungen dieser Reaktionsmedien.

Die Reduktion erfolgt durch Zugabe von 1 bis 2 Äquivalenten eines Hydriddonors wie z. B. Triethylsilan, Triphenylsilan, Tributylzinnhydrid, Natriumcyanoborhydrid, 1,4-Dihydronaphthalin oder 1,4-Cyclohexadien.

Die Reaktionstemperatur kann breit variiert werden, vorzugsweise arbeitet man jedoch zwischen 0 °C und 50 °C.

Die als Ausgangsstoffe verwendeten 1,4-Dihydropyridinderivate der alllgemeinen Formel (II) sind literaturbekannt oder können nach literaturbekannten Methoden hergestellt werden (vgl. z. B. K. Eisner und J. Kuthan, Chem. Rev. 72, 1 (1972); DOS 2 658 804).

Zusätzlich zu den Ausführungsbeispielen seien beispielhaft folgende Verbindungen genannt:

t-2,6-Dimethyl-t-4-(2-nitrophenyl)-1,2,3,4-tetrahydropyridin-r-3,5-dicarbonsäuredipropylester

t-2,6-Dimethyl-t-4-(3-nitrophenyl)-1,2,3,4-tetrahydropyridin-r-3,5-dicarbonsäuredipropylester

t-2,6-Dimethyl-t-4-(2-nitrophenyl)-1,2,3,4-tetrahydropyridin-r-3,5-dicarbonsäuredi(2-methoxyethyl)ester

t-2,6-Dimethyl-t-4-(3-nitrophenyl)-1,2,3,4-tetrahydropyridin-r-3,5-dicarbonsäuredi(2-methoxyethyl)ester

t-2,6-Dimethyl-t-4-(3-nitrophenyl)-1,2,3,4-tetrahydropyridin-r-3-carbonsäureisopropylester-5-carbonsäuremethylester

t-2,6-Dimethyl-t-4-(3-nitrophenyl)-1,2,3,4-tetrahydropyridin-r-3-carbonsäureethylester-5-carbonsäuremethylester

t-2,6-Dimethyl-t-4-(3-nitrophenyl)-1,2,3,4-tetrahydropyridin-r-3-carbonsäureisopropylester-5-carbonsäure-(2-methoxyethyl)ester

Je nach Art der Reste $R_1$, $R_2$, $R_3$, X und Y enthalten die erfindungsgemäßen Verbindungen mindestens drei Asymmetriezentren und können daher in mehreren stereoisomeren Formen auftreten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E. L. Eliel, Stereochemistry of Carbon Compounds, Mc Graw Hill, 1962).

Die neuen Verbindungen haben ein breites und vielseitiges pharmakologisches Wirkungsspektrum.

Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne einer Energieersparnis.
2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren. Die Verbindungen eignen sich daher besonders als Cerebraltherapeutika.
4. Die Verbindungen senken den Blutdruck von normotonen und hypertonen Tieren und können somit als antihypertensive Mittel verwendet werden.
5. Die Verbindungen haben stark muskulär-spasmolytische Wirkungen, die an der glatten Muskulatur des Magens, Darmtraktes, des Urogenitaltraktes und des Respirationssystems deutlich werden.

Die erfindungsgemäßen Verbindungen eignen sich aufgrund dieser Eigenschaften besonders zur Prophylaxe und Therapie der akuten und chronischen ischämischen Herzkrankheiten im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z. B. Erdölfraktionen), pflanzliche Öle (z. B. Erdnuß-/Sesam-Öl, Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzukker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin-Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger

Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß.

Ausführungsbeispiele

Beispiel 1

t-2,6-Dimethyl-t-4-(2-nitrophenyl)-1,2,3,4-tetrahydropyridin-r-3,5-dicarbonsäuredimethylester

10 g (29 mmol) 2,6-dimethyl-4-(2-nitrophenyl)-1,4-dihydropyridin-3,5-dicarbonsäuredimethylester werden in 100 ml Trifluoressigsäure gelöst und mit 5,6 ml (36,1 mmol) Triethylsilan versetzt. Anschließend wird die Reaktionsmischung 15 Minuten bei 50 °C gerührt. Danach wird mit viel Wasser verdünnt und 2 mal mit Essigester extrahiert. Die vereinigten Essigesterphasen werden 3 mal mit gesättigter Bicarbonatlösung gewaschen, mit Magnesiumsulfat getrocknet und eingedampft. Der so erhaltene Rückstand kristallisiert aus Ether. Ausbeute: 6,6 g (66% der Theorie); Fp.: 157–160 °C.

Die in der nachfolgenden Tabelle 1 aufgeführten Beispiele wurden analog zu Beispiel 1 hergestellt.

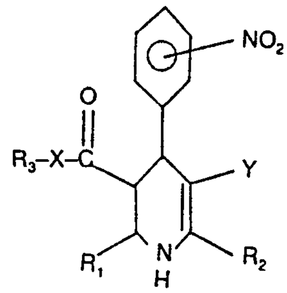

Tabelle 1

| Beispiel Nr. | $R_1$ | $R_2$ | $R_3$ | 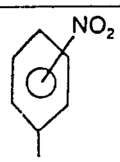 | X | Y | Schmelzpunkt (°C) | Ausbeute in % der Theorie |
|---|---|---|---|---|---|---|---|---|
| 2 | $C_2H_5$ | $C_2H_5$ | $CH_3$ | 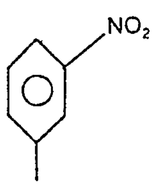 | O | $COOCH_3$ | 67–73 | 58 |
| 3 | $CH_3$ | $CH_3$ | $C_2H_5$ | 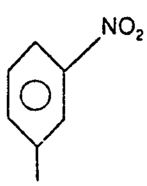 | O | $COOC_2H_5$ | 84–87 | 84 |
| 4 | $CH_3$ | $CH_3$ | $C_2H_5$ | 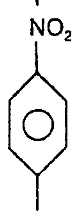 | O | $COOC_2H_5$ | 110–114 | 73 |
| 5 | $CH_3$ | $CH_3$ | $CH_3$ | 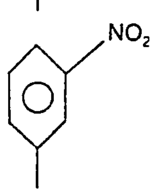 | Einfachbindung | 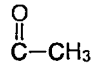 | 175–180 | 64 |

Tabelle 1 (Fortsetzung)

| Beispiel Nr. | $R_1$ | $R_2$ | $R_3$ | 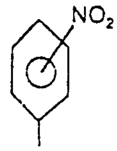 | X | Y | Schmelz- punkt (°C) | Ausbeute in % der Theorie |
|---|---|---|---|---|---|---|---|---|
| 6 | $CH_3$ | $CH_3$ | $CH_3$ | 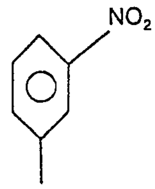 | O | CN | 202–204 | 63 |
| 7 | $CH_3$ | $CH_3$ | $C_2H_5$ | 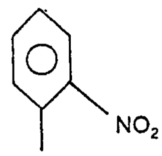 | O | $COOC_2H_5$ | 120 | 86 |

**Patentansprüche**

1. 4-(Nitrophenyl)-1,2,3,4-tetrahxdropyridine der allgemeinen Formel (I)

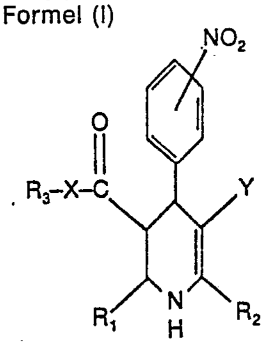

(I)

in welcher

$R_1$ und $R_2$ jeweils für Alkyl mit 1 bis 4 Kohlenstoffatomen stehen,

$R_3$ für einen geradkettigen oder verzweigten Alkylrest mit bis zu 10 Kohlenstoffatomen steht, der gegebenenfalls durch Sauerstoff in der Kette unterbrochen ist oder durch Fluor oder Chlor substituiert ist,

X für eine Einfachbindung oder ein Sauerstoffatom steht, und

Y für eine Nitrilgruppe oder den Rest $-CO-X'-R_4$ steht, wobei X' und $R_4$ jeweils die Bedeutung von X und $R_3$ besitzt und mit diesen identisch oder von diesen verschieden sein kann,

sowie ihre physiologisch unbedenklichen Salze.

2. Verfahren zur Herstellung von Verbindungen der allgemeinen Formel (I)

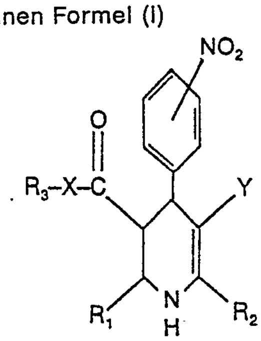

(I)

in welcher

$R_1$ und $R_2$ jeweils für Alkyl mit 1 bis 4 Kohlenstoffatomen stehen,

$R_3$ für einen geradkettigen oder verzweigten Alkylrest mit bis zu 10 Kohlenstoffatomen steht, der gegebenenfalls durch Sauerstoff in der Kette unterbrochen ist oder durch Fluor oder Chlor substituiert ist,

X für eine Einfachbindung oder ein Sauerstoffatom steht und

Y für eine Nitrilgruppe oder den Rest $-CO-X'-R_4$ steht, wobei X' und $R_4$ jeweils die Bedeutung von X und $R_3$ besitzt und mit diesen identisch oder von diesen verschieden sein kann,

dadurch gekennzeichnet, daß man Dihydropyridinverbindungen der allgemeinen Formel (II)

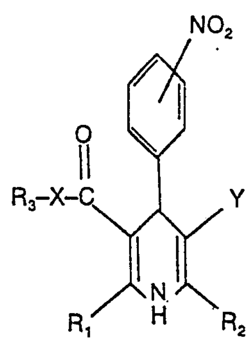

(II)

in welcher

$R_1$, $R_2$, $R_3$, Y und X die oben angegebene Bedeutung haben,

in einem stark sauren Reaktionsmedium mittels geeigneter Hydriddonoren reduziert.

3. Verfahren gemäß Anspruch 2, dadurch gekennzeichnet, daß man bei Temperaturen zwischen 0 °C und 50 °C arbeitet.

4. Verfahren gemäß Anspruch 2, dadurch gekennzeichnet, daß man als saures Reaktionsmedium starke organische Säuren oder Mischungen aus anorganischen oder organischen Säuren mit inerten organischen Lösungsmitteln verwendet.

5. Arzneimittel enthaltend mindestens eine Verbindung der allgemeinen Formel (I) gemäß Anspruch 1.

6. Verfahren zur Herstellung von Arzneimitteln, dadurch gekennzeichnet, daß man Verbindungen der allgemeinen Formel (I) gemäß Anspruch 1 gegebenenfalls unter Verwendung von Hilfs- und Trägerstoffen in eine geeignete Applikationsform überführt.

7. Verwendung von Verbindungen der allgemeinen Formel (I) gemäß Anspruch 1 bei der Herstellung von kreislaufwirksamen Arzneimitteln.

**Claims**

1. 4-(Nitrophenyl)-1,2,3,4-tetrahydropyridines of the general formula (I)

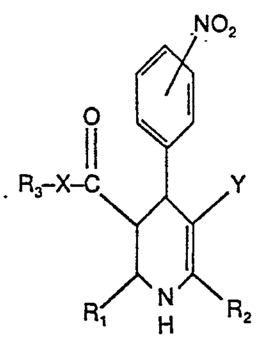

(I)

in which

$R_1$ and $R_2$ each represent alkyl with 1 to 4 carbon atoms,

$R_3$ represents a straight-chain or branched alkyl radical which has up to 10 carbon atoms and is optionally interrupted by oxygen in the chain or substituted by fluorine or chlorine,

X represents a single bond or an oxygen atom and

Y represents a nitrile group or the radical $-CO-X'-R_4$ wherein

X' and $R_4$ in each case have the meaning of X and $R_3$ and can be identical to or different from these radicals,

and their physiologically acceptable salts.

2. Process for the preparation of compounds of the general formula (I)

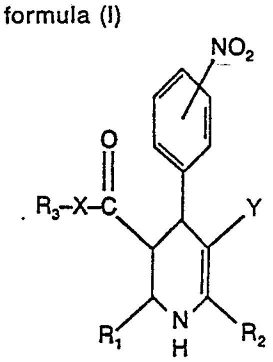

(I)

in which

$R_1$ and $R_2$ each represent alkyl with 1 to 4 carbon atoms,

$R_3$ represents a straight-chain or branched alkyl radical which has up to 10 carbon atoms and is optionally interrupted by oxygen in the chain or substituted by fluorine or chlorine,

X represents a single bond or an oxygen atom and

Y represents a nitrile group or the radical $-CO-X'-R_4$ wherein

X' and $R_4$ in each case have the meaning of X and $R_3$ and can be identical to or different from these radicals,

characterised in that dihydropyridine compounds of the general formula (II)

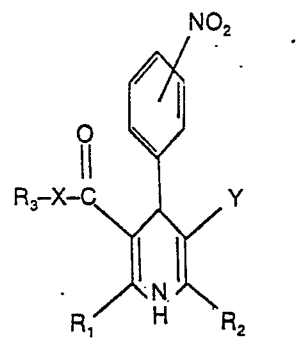

(II)

in which

$R_1$, $R_2$, $R_3$, Y and X have the abovementioned meaning,

are reduced in a strongly acid reaction medium by means of suitable hydride donors.

3. Process according to Claim 2, characterised in that the reaction is carried out at temperatures between 0 °C and 50 °C.

4. Process according to Claim 2, characterised in that strong organic acids or mixtures of inorganic or organic acids with inert organic solvents are used as the acid reaction medium.

5. Medicament containing at least one compound of the general formula (I) according to Claim 1.

6. Process for the preparation of medicaments, characterised in that compounds of the general formula (I) according to Claim 1 are converted into a suitable administration form, if appropriate using auxiliaries and excipients.

7. Use of compounds of the general formula (I) according to Claim 1 in the preparation of medicaments which act on the circulation.

**Revendications**

1. 4-(nitrophényl)-1,2,3,4-tétrahydropyridines de formule générale (I):

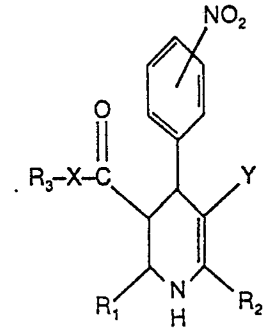

(I)

dans laquelle

$R^1$ et $R^2$ représentent chacun un radical alkyle contenant 1 à 4 atomes de carbone,

$R^3$ représente un radical alkyle à chaîne droite ou ramifiée contenant jusqu'à 10 atomes de carbone et éventuellement interrompu dans sa chaîne par un atome d'oxygène, ou substitué par un atome de fluor ou un atome de chlore,

X représente une liaison simple ou un atome d'oxygène, et

Y représente un groupe nitrile ou le radical $-CO-X'-R^4$, X' et $R^4$ ayant chacun la signification de X et $R^3$ et pouvant être identiques à ou différents de ceux-ci,

ainsi que leurs sels physiologiquement inoffensifs.

2. Procédé de préparation de composés de formule générale (I):

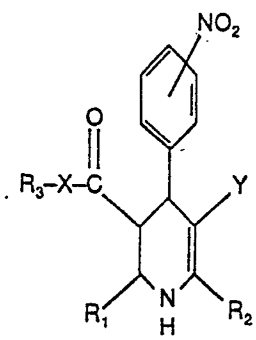

(I)

dans laquelle

$R^1$ et $R^2$ représentent chacun un radical alkyle contenant 1 à 4 atomes de carbone,

$R^3$ représente un radical alkyle à chaîne droite ou ramifiée contenant jusqu'à 10 atomes de carbone, éventuellement interrompu dans sa chaîne par un atome d'oxygène, ou substitué par un atome de fluor ou un atome de chlore,

X représente une liaison simple ou un atome d'oxygène, et

Y représente un groupe nitrile ou le radical $-CO-X'-R^4$, X' et $R^4$ ayant chacun la signification de X et $R^3$ et pouvant être identiques à ou différents de ceux-ci,

caractérisé en ce qu'on réduit des composés de dihydropyridines de formule générale (II):

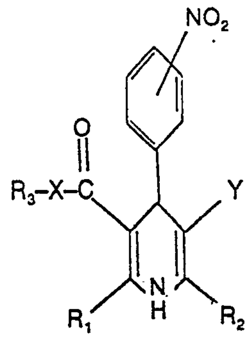

(II)

dans laquelle

$R^1$, $R^2$, $R^3$, Y et X ont les significations indiquées ci-dessus,

dans un milieu réactionnel fortement acide, au moyen de donneurs d'hydrures appropriés.

3. Procédé selon la revendication 2, caractérisé en ce qu'on travaille à des températures comprises entre 0 et 50 °C.

4. Procédé selon la revendication 2, caractérisé en ce que, comme milieu réactionnel acide, on emploie des acides organiques forts ou des mélanges d'acides inorganiques ou organiques avec des solvants organiques inertes.

5. Médicament contenant au moins un composé de formule générale (I) selon la revendication 1.

6. Procédé de préparation de médicaments, caractérisé en ce qu'on transforme des composés de formule générale (I) selon la revendication 1, sous une forme d'application appropriée en utilisant éventuellement des substances auxiliaires et des substances supports.

7. Utilisation de composés de formule générale (I) selon la revendication 1, lors de la préparation de médicaments agissant sur la circulation.